# EUROPÄISCHE PATENTSCHRIFT

(11) **EP 0 603 423 B1**
(45) Veröffentlichungstag und Bekanntmachung des Hinweises auf die Patenterteilung: **10.09.1997**
(21) Anmeldenummer: 92121677.6
(22) Anmeldetag: 20.12.1992
(51) Int. Cl.: A61F 2/06

(54) **Gerät zum Implantieren einer Endoprothese**
Device for the implantation of an endoprothesis
Dispositif pour l'implantation d'une endoprothese

(43) Veröffentlichungstag der Anmeldung: 29.06.1994
(73) Patentinhaber: SCHNEIDER (EUROPE) AG, 8180 Bülach (CH)
(72) Erfinder: Gianotti, Marc, CH-8542 Wiesendangen (CH); Baldinger, Rolf, CH-5444 Künten (CH); Hank, Susanne, CH-5612 Villmergen (CH)
(74) Vertreter: Blum, Rudolf Emil Ernst

(56) Entgegenhaltungen:
- EP-A- 0 518 838
- US-A- 4 557 255
- US-A- 4 665 918
- US-A- 5 160 341

## Beschreibung

Die vorliegende Erfindung betrifft ein Gerät zum Implantieren einer Endoprothese in ein kanalförmiges Gefäss des menschlichen oder tierischen Körpers wie in den Ansprüchen 1 bis 6 definiert wird.

Implantierte Endoprothesen kommen allgemein bei Lumenverengungen, beispielsweise bei Verengungen von Blutgefässen zum Einsatz. Diese Endoprothesen, auch Stents genannt, sind oft Drahtgeflechtsrohre, die aus derart elastischen Filamenten aufgebaut sind, dass das Drahtgeflechtsrohr in einem radial komprimierten Zustand mittels einem Implantiergerät an die aufzuweitende Stelle des zu behandelnden Gefässes gebracht wird, wonach das Implantiergerät die Endoprothese freigibt, so dass dieselbe sich aufweiten, an die Wände des Gefässes anlegen und die Verengung aufweiten kann.

Solche implantierbare Endoprothesen und entsprechende Geräte zum Implantieren derselben sind in der US-A-4,655,771; US-A-4,954,126; US-A-5,026,377 offenbart. Eine weitere Offenbarung ist zu finden im Buch "Katheterinterventionen in der Angiologie" von Felix Mahler, Georg Thieme Verlag Stuttgart, New York, auf Seiten 31-35 (allgemeine Methodik und Instrumentation). Es wird aus Gründen der Offenbarung ausdrücklich auf diese Schriftwerke verwiesen.

Das Drahtgeflechtsrohr oder eine entsprechend aufgebaute Endoprothese ist dabei im radial komprimierten Zustand in einem äusseren Hülsenglied eingespannt. Im Inneren des Hülsengliedes liegt ein mit einer Schulter versehenes, als stationär festgehaltener Stössel ausgebildetes Kernglied. Das Kernglied ist axial relativ zur äusseren Hülse verschiebbar und kann mit seiner Schulter an der Endoprothese anliegen. Zur Freigabe der Endoprothese wird eine Relativbewegung des Hülsengliedes gegenüber dem Kernglied durchgeführt. Bei der richtigen Bedienung des Implantiergerätes wird für diese Relativbewegung das Kernglied stationär gehalten und das Hülsenglied wird zurückgezogen.

Wenn das Implantiergerät falsch bedient wird und zur Ausführung der Relativbewegung nicht das Kernglied sondern das Hülsenglied stationär gehalten wird, dann tritt zwar die Endoprothese am distalen Ende des Hülsengliedes ordnungsgemäss aus und die Endoprothese wird freigegeben, die Endoprothese wird bei dieser Prozedur jedoch im Gefäss vorgeschoben und das Gefäss wird dadurch verletzt. Ohne dass der Arzt eine Möglichkeit hätte, dies in Echtzeit-Bildern optisch zu kontrollieren, bohren sich dabei die freien Enden der Endoprothese in die Gefässwand. Gleichzeitig können die für diese Belastung nicht ausgelegten Elemente der Endoprothese, also beispielsweise die erwähnten elastischen Filamente, sich bleibend verformen und die Endoprothese auf diese Weise unwirksam machen. Es sind zwar schon Implantiergeräte vorgeschlagen worden, die eine Rückfaltung der Endoprothese wieder zurück in das äussere Hülsenglied erlauben. Wenn jedoch die Elemente der Endoprothese durch diese Fehlbedienung bleibend verformt sind, ist selbst dieser Ausweg nicht mehr sicher. Die verbogene und damit unwirksam gewordene Endoprothese muss also gegebenenfalls im Gefäss zurückbleiben. Auch der Lageort der unwirksam gewordenen Endoprothese kann nicht mehr verändert werden. Sie kann höchstens noch, solange sie nur teilweise entfaltet ist, in engen Grenzen zur Bedienungsseite hin gezogen werden. Im ungünstigsten Fall muss daher eine unbrauchbar gewordene Endoprothese genau an dem Ort im Gefäss zurückbleiben, an dem an sich eine intakte Endoprothese indiziert war, das heisst, an dem Ort, an dem das Gefäss krankhaft verändert ist.

Eventuelle Fehlbedienungen der Implantiergeräte für diese Endoprothesen können also zu Situationen führen, die weitreichende Konsequenzen haben. Fehlbedienungen können sich bei diesen Geräten in jedem Fall schon deshalb leicht einstellen, weil die äussere Hülse, die bei der richtigen Bedienung zurückgezogen werden muss, auf fast ihrer ganzen Länge im Gefäss gelagert ist und dort mit einer gewissen Haftreibung gehalten wird. Das äussere Hülsenglied widersetzt sich daher der zur richtigen Bedienung notwendigen Bewegung mit einem gewissen Widerstand.

Bei den bekannten Geräten ist zwar jeweils das bedienungsseitige Ende so ausgebildet, dass die Relativbewegung zwischen Kernglied und äusserem Hülsenglied leicht und sicher in einer Scherenbewegung durchgeführt werden kann. Die bekannten Geräte weisen jedoch den Nachteil auf, dass die Betätigungsgriffe in einer Weise gestaltet sind, bei der beide Bewegungstypen, der richtige Bewegungstyp mit stationärem Kernglied und der falsche Bewegungstyp mit stationärem Hülsenglied, vollkommen gleichwertig nebeneinander zugelassen sind. In bezug auf die richtige oder falsche Bedienung entsteht durch die bekannten Griffanordnungen keine bevorzugte Handhabung. Die durch die bekannten Griffe nahegelegte Scherenbewegung beinhaltet zwar die eine einzige richtige, daneben aber auch eine Vielzahl von falschen Bewegungsformen. Die Auswahl zwischen richtiger und falscher Bedienung ist bei den bekannten Griffen allein in den Bereich der Verstandestätigkeit des Arztes gelegt, der tatsächlich bei der Bedienung dann durchgeführte Bewegungsablauf ist abhängig von den Fähigkeiten des Arztes, das Ergebnis der vorherigen Auswahl dann auch zuverlässig in die Praxis umzusetzen. Dazu kommt noch, dass die Griffanordnung der bekannten Geräte von einzelnen Bedienern mit der Betätigungsanordnung einer herkömmlichen Injektionsspritze assoziiert werden kann. Bei herkömmlichen Injektionsspritzen wird normalerweise der Kolben gegenüber dem aussen liegenden Zylinder vorgeschoben. Genau diese Bewegung ist aber hier die falsche Bewegung.

Ausserdem hat es sich gezeigt, dass in manchen Anwendungsfällen die bekannten Griffe unvollkommen sind, wenn es darum geht, das Implantiergerät mit der notwendigen Zuverlässigkeit und Sicherheit in die richtige Ausgangsposition vor der Freigabe der Endoprothese zu plazieren.

Die US-A-4 665 918 offenbart ein Gerät zum Implantieren einer Endoprothese, welches ein Kernglied aufweist, auf welchem die Endoprothese in einem zusammengedrückten Zustand aufzusetzen ist. Dieses Kernglied ist in einem ersten Hülsenglied längsverschiebbar angeordnet das dazu dient, die Endoprothese im zusammengedrückten Zustand zu halten. Wird das erste Hülsenglied relativ zum ersten Kernglied zurückgezogen, wird die Endoprothese freigegeben. Das erste Hülsenglied ist seinerseits in einem zweiten Hülsenglied längsverschiebbar angeordnet. Zum Einsetzen einer Endoprothese werden die drei genannten Bauteile in einem teleskopförmig zusammengeschobenen Zustand bis unmittelbar vor die zu erweiternde Stelle in einem betreffenden Gefäss eingeführt. Danach werden das erste Hülsenglied und das Kernglied zusammen um eine kurze Strecke aus dem zweiten Hülsenglied hinausgeschoben, und schlussendlich wird zum Freigeben der Endoprothese das Kernglied aus dem ersten Hülsenglied hinausgeschoben. Zur Bedienung dieses Gerätes sind sehr viele unbequem zu betätigende Griffe erforderlich, die nur mit Fingern ergriffen werden können, wobei kein Griff vorhanden ist, mittels welchem das Gerät zuverlässig in eine Ausgangsposition zur Freigabe der Endoprothese geschoben und dort sicher bei der betreffenden Stelle unbewegbar gehalten werden kann.

Hier will die Erfindung Abhilfe schaffen. Die Erfindung, wie sie in den Ansprüchen gekennzeichnet ist, löst die Aufgabe, ein Gerät zum Implantieren einer Endoprothese in ein kanalförmiges Gefäss des menschlichen oder tierischen Körpers zu schaffen, das einfach herzustellen ist, dessen Plazierung an die Ausgangsposition vor der Freigabe der Endoprothese erleichtert ist, das geringe Anforderungen an die Geschicklichkeit der Bedienungsperson stellt und bei dem die richtige Bedienung des Gerätes erleichtert, die falsche Bedienung dagegen erschwert ist.

Durch die Erfindung, wie sie in Anspruch 1 gekennzeichnet ist, wird erreicht, dass das Implantiergerät mit der notwendigen Zuverlässigkeit und Sicherheit in die richtige Ausgangsposition vor der Freigabe der Endoprothese plaziert werden kann. Das äussere Hülsenglied ist durch die Arretiervorrichtung gegenüber dem Kernglied gesichert. Es muss nicht mehr das äussere Hülsenglied zum Setzen des Implantiergerätes manipuliert werden, sondern es kann das am proximalen Ende des Gerätes weiter herausragende innere Kernglied zur Manipulation des gesamten Gerätes herangezogen werden. An diesem inneren Kernglied ist jetzt ein zum Ergreifen mit dem Daumen und dem ersten Glied des Zeigefingers ausgebildeter abstehender pistolengrifförmiger Handgriff angeordnet. Durch die abstehende Pistolengrifform, die in der angegebenen Form ergriffen werden kann, wird sowohl die Steuerung als auch der Vorschub des Implantiergerätes vom Handgelenk der Bedienungsperson kontrolliert. Bei den hier auftretenden Kräften ist das eine sichere, zuverlässige und genaue Kontrolle, die vom Arzt weniger Fingerfertigkeit verlangt.

Am äusseren Hülsenglied ist demgegenüber kein Pistolengriff, sondern ein Handgriff wie bei den bekannten Geräten vorgesehen. Dieser andere Grifftyp dient lediglich als Widerlager für einen einseitig angreifenden, den Griff nicht umschliessenden Finger. Durch den am inneren Kernglied angeordneten Pistolengriff und durch die Tatsache, dass am anderen Teil ein ganz anderer Griff angeordnet ist, wird das innere Kernglied immer dem leicht stationär zu haltenden Handgelenk zugeordnet. Das innere Kernglied wird nicht etwa den Fingern oder einem Finger zugeordnet.

Bei einer Scherenbewegung zwischen Daumen und Zeigefinger wie beim Stand der Technik, erfordert es vielleicht nur ein geringes Mass an Fingergelenkigkeit und Geschicklichkeit, den Daumen stationär zu halten und den Zeigefinger auf den Daumen zu zu bewegen. Es erfordert aber eine relativ grosse Geschicklichkeit, bei dieser Bewegung im Stand der Technik den Zeigefinger stationär zu halten und den Daumen auf den Zeigefinger zu zu bewegen. Die Erfindung schlägt demgegenüber zur Bedienung des Gerätes einen ganz anderen Weg ein. Entweder, es wird der oder es werden die Finger auf das Handgelenk zu bewegt, wenn der Griff an der äusseren Hülse vom Pistolengriff aus nämlich in Reichweite der Finger ist, oder es wird für die Scherenbewegung die zweite Hand eingesetzt. Im ersten Fall handelt es sich um eine Greifbewegung und damit die einfachste denkbare Bedienungsbewegung überhaupt. Sie erfordert das geringste Mass an Geschicklichkeit, Fingergelenkigkeit und Aufmerksamkeit. Im zweiten Fall, wenn der Griff an der äusseren Hülse vom Pistolengriff aus ausserhalb der Reichweite der Finger ist, führt die zweite Hand allein die Scherenbewegung aus, ohne Rücksicht auf stationäres Halten. Das Handgelenk, dem in beiden Fällen jeweils der Pistolengriff zugeordnet ist, lässt sich bei dieser Operation wie selbstverständlich stationär halten, weil seine Position von einer ganz anderen Muskelapparatur gesteuert wird als die Finger. Durch die Merkmalskombination der Erfindung wird die richtige Bedienung daher erleichtert, sie erfordert vom Arzt keine besondere Gelenkigkeit mehr. Der Bewegungswiderstand des äusseren Hülsengliedes im Gefäss kann somit leichter überwunden werden, ohne gleichzeitig eine schiebende Gegenkraft auf das Kernglied auszuüben.

Die Griffe der äusseren Hülse und des inneren Kerns sind nach der Erfindung stark unterschiedlich ausgebildet und zwar jeweils für eine unterschiedliche Form der Ergreifung. Die falsche Bedienung des Gerätes wird dadurch entscheidend erschwert, die Griffe müssten dazu in einer Form ergriffen werden, für die sie nicht ausgebildet sind. Das bedeutet aber, dass die Auswahl zwischen richtiger und falscher Bedienung nicht allein der Verstandestätigkeit des Arztes überlassen ist. Bei der erfindungsgemässen Anordnung müssten, bevor die falsche Bedienung tatsächlich zur Ausführung kommen kann, bei der falschen Auswahl auch noch deutliche Hürden in der Manipulation überwunden werden. Eine unwillkürliche falsche Bedienung ist daher praktisch ausgeschlossen.

Der in der Merkmalskombination der Erfindung vorgesehene Pistolengriff ist aus vielen Lebensbereichen bekannt und er wird in der Erfindung genau für die Bedienungsform eingesetzt, für die er auch aus den anderen Bereichen bekannt ist. Die Merkmalskombination der Erfindung legt daher auch dem unvoreingenommenen Betrachter die Bedienungsform nahe, insbesondere kann die Ausbildung nach der Erfindung keine falschen Assoziationen hervorrufen, die eine falsche Bedienung zur Folge haben können.

Ein weiterer Vorteil ergibt sich daraus, dass durch die Arretierung des äusseren Hülsengliedes am Kernglied einerseits und den Pistolengriff andererseits ein Implantiergerät entsteht, das beim Vorschieben des Gerätes im Gefäss besonders feinfühlig gedreht werden kann. Mit einem an sich bekannten schrägen Anschnitt an der Spitze ist dieses Gerät dann ohne Dilator plazierbar. Das heisst, es kann ohne eine separate Einheit gebraucht werden, die, z.B. kegelförmig, das Gefäss zunächst aufweitet, bevor das Gerät vorgeschoben wird. Der schräge Anschnitt an der Spitze des Gerätes kann mit einer vorsichtigen Drehung des Gerätes am Pistolengriff durch etwa vorhandene Engstellen im Gefäss hindurch geführt werden. In der Luftröhre sind solche Engstellen z.B. die Stimmbänder.

Durch den an dem Kernglied zusätzlich zum pistolengrifförmigen Handgriff vorgesehenen weiteren Handgriff, der als Widerlager für einen einseitig angreifenden, den Griff nicht umschliessenden Finger ausgebildet ist und dem Griff des Hülsengliedes gegenüberliegt, kann das Gerät leichter mit zwei Händen bedient werden. Eine Hand hat nur die Aufgabe, die Relativbewegung zwischen dem Hülsenglied und dem Kernglied herbeizuführen, die andere Hand steht ohne Nebenaufgaben allein zum stationären Halten des Geräts zur Verfügung. In diesem Fall ist zur Herbeiführung der Relativbewegung die ganze Handspanne einsatzfähig, es kann daher ohne Uebersetzung in direkter Auslösung eine besonders lange Endoprothese freigesetzt werden. Der Pistolengriff kann dabei besonders wirkungsvoll nicht nur vom Daumen und dem ersten Glied des Zeigefingers ergriffen werden, sondern er kann in diesem Fall von der ganzen Hand umschlossen werden. Mit dieser Kombination von Merkmalen ist auch ein Gerät denkbar, dessen Bedienung mit nur einer Hand unmöglich wird, sobald der Pistolengriff vom Bediener erfasst ist. Dazu muss nur der am äusseren Hülsenglied angebrachte Griff in ausreichender Entfernung vom Pistolengriff angeordnet werden. Wegen der Trennung der Funktionen "stationär Halten" and "Freisetzen" ist die Bedienung mit zwei Händen bei langen Freisetzungsbewegungen auf jeden Fall eine Methode mit dem Vorteil grosser Zuverlässigkeit.

Eine besonders feinfühlige Positionierung des Gerätes in die Ausgangslage zum Freisetzen der Endoprothese und beim Freisetzen ein besonders zuverlässiges Halten in stationärer Position wird möglich, wenn der pistolenförmige Handgriff am Kernglied zum Umschliessen mit Mittel-, Ring- und kleinem Finger ausgebildet ist.

Dadurch, daß der Betätigungsgriff des Hülsengliedes zwei, einander diametral gegenüberliegende Vorsprünge aufweist, der weitere Handgriff des Kerngliedes dem pistolengrifförmigen Handgriff diametral gegenüberliegt, und die zwei Vorsprünge des Hülsengliedes in einer Ebene liegen, die mit der vom pistolengrifförmigen Handgriff und dem weiteren Handgriff bestimmten Ebene zusammenfällt, wird eine Haltung des Bedieners ermöglicht, die der menschlichen Anatomie sehr weit entgegen kommt. Es entstehen keine starken Abwinklungen an den Handgelenken und die Unterarme des Bedieners stehen bei der Bedienung des Gerätes ungefähr im Winkel von 90 Grad zueinander. Weil Extremlagen der Gliedmassen des Bedieners vermieden werden, ist damit ein hohes Mass an Sicherheit und Zuverlässigkeit bei der Bedienung gewährleistet.

Im folgenden wird die Erfindung anhand von lediglich einen Ausführungsweg darstellenden Zeichnungen näher erläutert.

Es zeigt:

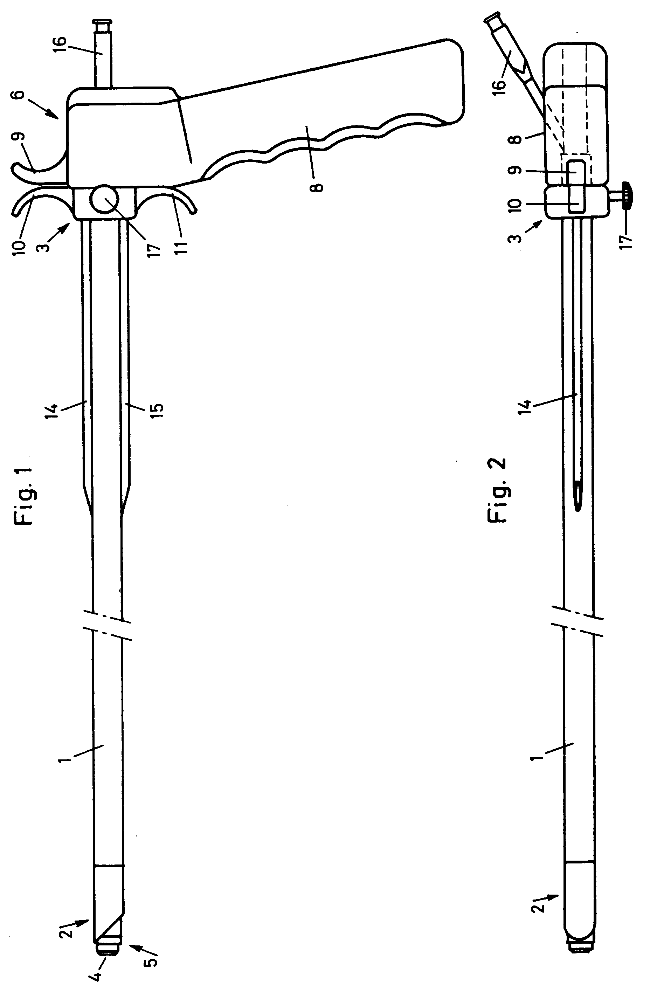

Figur 1 eine Seitenansicht eines erfindungsgemäss ausgebildeten Implantiergerätes,
Figur 2 eine Aufsicht auf das Gerät nach Figur 1,

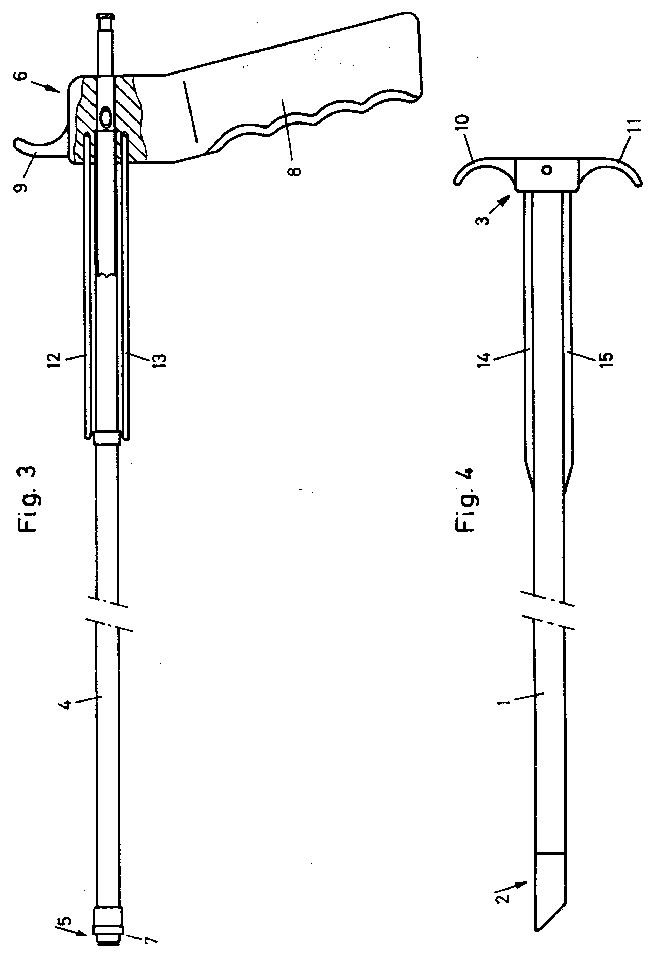

Figur 3 eine Seitenansicht des Kerngliedes des Gerätes, und
Figur 4 eine Seitenansicht des Hülsengliedes des Gerätes.

Das Implantiergerät ist grundsätzlich aus zwei Teilen, einem äusseren Hülsenglied 1 und einem inneren Kernglied 4 zusammengesetzt. Diese zwei Glieder sind relativ zueinander verschiebbar. Das distale Ende 2 des Hülsengliedes 1 ist auseinanderlaufend und weiter abgeschrägt ausgebildet. In dieses distale Ende 2 wird die Endoprothese auf eine gewünschte Länge geschnitten in einem elastisch zusammengepressten Zustand eingesetzt und gehalten. Beim proximalen Ende 3 des Hülsengliedes 1 sind zwei Vorsprünge 10, 11 vorhanden und es ist ersichtlich, dass eine Zurückziehbewegung des Hülsengliedes 1 relativ zum Kernglied 4, auf welche noch eingegangen wird, ausschliesslich durch diese Vorsprünge 10, 11 umgreifende Finger, z.B. Zeigefinger und der Mittelfinger der menschlichen Hand ermöglicht ist.

Beim distalen Ende 5 des Kerngliedes 4 ist eine Schulter 7 ausgebildet. Somit ist das Kernglied 4 als stationär gehaltener Stössel ausgebildet, welcher auf eine Stirnseite einer Endoprothese einwirken kann. Das Kernglied 4 weist beim proximalen Ende 6 einen pistolengrifförmigen Handgriff 8 auf. Diesem diametral gegenüberliegend ist ein weiterer, als Widerlager wirkender Griff 9 vorhanden. Es ist ersichtlich, dass der pistolengrifförmigen Handgriff 8, der weitere Griff 9, der Vorsprung 10 und der Vorsprung 11 alle in derselben Ebene verlaufen.

Vom Bereich des pistolengrifförmigen Handgriffes 8 des Kerngliedes 4 ragen zwei Führungsstangen 12, 13 in Richtung des distalen Endes des Kerngliedes 4. Diese Führungsstangen 12, 13 sind in Rohrstücken 14, 15 des Hülsengliedes 1 eingesetzt, so dass eine relative Rotationsbewegung zwischen Hülsenglied 1 und Kernglied 4 verunmöglicht ist, womit die genannten Handgriffe bzw. Vorsprünge ihre vorgegebene Stellung relativ zueinander immer einhalten werden. Der Innenraum des Kerngliedes 4 steht über einen Anschluss 16 mit der Umgebung in Verbindung. Durch diesen Anschluss kann beispielsweise ein Medium in das zu behandelnde Gefäss eingebracht werden oder nötigenfalls eine Absaugung durchgeführt werden.

Das Kernglied 4, das auf Grund des Innenraumes hohlzylindrisch ausgebildet ist, kann auch im Innenraum weitere medizinische Instrumente aufnehmen. Beispielsweise kann im Kernglied 4 ein Endoskop angeordnet werden. Damit kann die Plazierung der Endoprothese unter Sicht stattfinden. Wieder würde die Verbindung von aussen durch den Anschluss 16 hindurch verlaufen.

Das Hülsenglied 1 weist weiter beim proximalen Ende 3 eine Arretierschraube 17 auf, mittels welcher das Hülsenglied 1 auf dem Kernglied 4 arretiert werden kann.

Die Handhabung des Gerätes ist wie folgt. Als erster Schritt wird das Hülsenglied 1 auf dem Kernglied 4 in den Figuren nach links vorgeschoben, so dass die Schulter 7 des Kerngliedes 4 innerhalb des Hülsengliedes 1 zu liegen kommt. Danach wird das Hülsenglied 1 auf dem Kernglied 4 durch ein Anziehen der Arretierschraube 17 arretiert. Danach wird die Endoprothese beim distalen Ende 2 in das Hülsenglied 1 im elastisch zusammengedrückten Zustand eingeführt, so dass sie bei einer Stirnseite der Schulter 7 des Kerngliedes 4 gegenüberliegt. Eine Berührung ist nicht unbedingt notwendig, da die Endoprothese aufgrund ihrer elastischen Vorspannkraft gegen die Innenwand des Hülsengliedes 1 anliegt und somit gegen ein ungewolltes Verschieben gehalten ist. Zum Einbringen der Endoprothese in das zu behandelnde Gefäss, wobei sämtliche vorausgehenden medizinischen Massnahmen gemäss allgemeinen bekannten Vorgehen vorgenommen worden sind, ergreift der Arzt mindestens mit dem Daumen und dem ersten Glied des Zeigefingers den pistolengrifförmigen Handgriff 8. Das Implantiergerät mit der Endoprothese wird danach in das Gefäss eingeführt bis die Endoprothese genau bei der erwünschten Stelle im Gefäss angeordnet ist. Die Vorgehen zum Ueberwachen und Kontrollieren der Stelle solcher Endoprothesen sind ebenfalls allgemein bekannt. Es ist nun offensichtlich, dass aufgrund des pistolengrifförmigen Handgriffes ein äusserst sicheres Manipulieren des Gerätes, nötigenfalls Rotieren ermöglicht ist.

Zum Freigeben der Endoprothese wird vorerst durch die andere Hand die Arretierschraube 17 gelöst und danach kann durch Anlegen von zwei Fingern der anderen Hand an die Vorsprünge 10 und 11 und durch Anlegen des Daumens dieser Hand an den widerlagerförmigen Vorsprung 9 das Hülsenglied 1 langsam in Richtung gegen den pistolengrifförmigen Handgriff 8 zurückgezogen werden. Da der Handgriff 8 grundsätzlich immer noch von der Faust umschlossen ist, ist die Krafteinwirklung auf die Vorsprünge 10, 11 und somit auf das Hülsenglied 1 kleiner als die Krafteinwirkung auf das Kernglied 4, dass nun unverrückbar gehalten werden muss, so dass eine ungewollte Verschiebung der Endoprothese während dieser Manipulation nicht möglich ist. Durch Anliegen an der Schulter 7 des Kerngliedes 4 wird durch das Zurückziehen des Hülsengliedes 1 die Endoprothese aus dem Innenraum des Hülsengliedes 1 austreten und kann sich nun entspannend erweiteren, an der Innenwand des zu behandelnden Gefässes anliegen und dort seine aufweitende Funktion erfüllen.

Es ist also ersichtlich, dass die menschliche Krafteinwirkung auf denjenigen Teil des Gerätes, der bei der Manipulation zur Freigabe der Endoprothese nicht bewegt werden darf, viel grösser ist, als die auf den zu verschiebenden Teil des Gerätes, womit ein sicheres Setzen der Endoprothese möglich ist, ohne dass Fehlmanipulationen auftreten können.

Abschliessend soll noch festgehalten werden, dass das Implantieren in Sinne dieser Erfindung auch ein vorübergehendes Plazieren einer Endoprothese in einem Gefäss beinhaltet.

## Patentansprüche

1. Gerät zum Implantieren einer Endoprothese in ein kanalförmiges Gefäss des menschlichen oder tierischen Körpers, mit einem langgestreckten, rohrförmigen Hülsenglied (1), das ein distales (2) und ein proximales Ende (3) aufweist, welches distale Ende (2) zur Aufnahme einer darin in einem radial komprimierten Zustand einzusetzenden Endoprothese ausgebildet ist, und mit einem langgestreckten, im äusseren Hülsenglied (1) relativ zu demselben verschiebbaren und dazu koaxial angeordneten, als stationärer Stössel ausgebildetes Kernglied (4), das ein distales (5) und ein proximales Ende (6) aufweist, wobei beim Bereich des distalen Endes (5) eine zur Anlage an eine eingesetzte Endoprothese bestimmte Schulter (7) angeordnet ist, derart, dass eine im Hülsenglied (1) eingesetzte Endoprothese durch eine Relativbewegung des Hülsengliedes (1) gegenüber dem Kernglied (4) ausgebbar ist, wodurch die Endoprothese selbsttätig expandiert und sich an die Wand des Gefässes anlegt, welches Hülsenglied (1) und welches Kernglied (4) beim proximalen Ende (3, bzw. 6) jeweils einen Betätigungsgriff aufweisen, welches Gerät eine Vorrichtung (17) zum Arretieren des äusseren Hülsengliedes gegenüber dem Kernglied aufweist und der Betätigungsgriff des Kerngliedes als ein zum Ergreifen mit dem Daumen und dem ersten Glied des Zeigefingers einer menschlichen Hand ausgebildeter, abstehender pistolengrifförmiger Handgriff (8) zum Halten des Implantiergerätes bei der Plazierung der Endoprothese im kanalförmigen Gefäss ausgebildet ist, welcher Betätigungsgriff des Hülsengliedes (1) zwei, einander diametral gegenüberliegende, abstehende Vorsprünge (10, 11), die als Widerlager für einen einseitig angreifenden, den Griff nicht umschliessenden menschlichen Finger ausgebildet sind aufweist und ein weiterer Handgriff (9) des Kerngliedes (4) dem pistolengrifförmigen Handgriff (8) diametral gegenüberliegt, wobei die zwei Vorsprünge (10, 11) des Hülsengliedes (1) in einer Ebene liegen, die mit der vom pistolengrifförmigen Handgriff (8) und dem weiteren Handgriff (9) bestimmten Ebene zusammenfällt.

2. Gerät nach Anspruch 1, dadurch gekennzeichnet, dass der weitere Handgriff (9) als Widerlager für einen einseitig angreifenden, den Griff nicht umschliessenden menschlichen Finger ausgebildet ist.

3. Gerät nach Anspruch 1 oder 2, dadurch gekennzeichnet, dass der pistolengrifförmige Handgriff (8) am Kernglied (4) zum Umschliessen mit Mittel-, Ring- und kleinen Finger einer menschlichen Hand ausgebildet ist.

4. Gerät nach einem der Ansprüche 1 - 3, dadurch gekennzeichnet, dass das Kernglied (4) beim proximalen Ende mindestens eine gegen ihr distales Ende gerichtete Führungsstange (12, 13) aufweist, die in ein mit dem Hülsenglied (1) fest verbundenes Rohrstück (14, 15) geführt hineinragt, um eine relative Rotationsbewegung zwischen dem Hülsenglied (1) und dem Kernglied (4) zu verhindern.

5. Gerät nach einem der Ansprüche 1 - 4, dadurch gekennzeichnet, dass das Kernglied (4) und das Hülsenglied (1) aus einem starren Werkstoff bestehen.

6. Gerät nach einem der Ansprüche 1 - 5, dadurch gekennzeichnet, dass das Kernglied (4) zur Aufnahme weiterer medizinischer Instrumente hohlzylindrisch ausgebildet ist.

## Claims

1. Apparatus for the implantation of an endoprothesis into a channel shaped vessel of the human or animal body, with an elongate, tube shaped sleeve member (1) which includes a distal (2) and a proximal end (3), which distal end (2) is designed for receipt of an endoprothesis to be inserted thereinto in a radially compressed state, and with an elongate core member (4) which is arranged in the outer core member (4) displaceably and coaxially thereto and is structured as a stationary plunger, and includes a distal (5) and a proximal end (6), whereby a shoulder (7) is located at the area of the distal end (5) and adapted for contacting an inserted endoprothesis, such that an endoprothesis inserted into the sleeve member (1) is dispensable by a relative movement of the sleeve member (1) in relation to the core member (4), wherewith the endoprothesis expands automatically and comes to rest against the wall of the vessel, which sleeve member (1) and which core member (4) both include a respective operating handle at the proximal end (3 or 4, resp.), which apparatus includes a device (17) for a locking of the outer sleeve member relative to the core member and the operating handle of the core member is shaped as a projecting pistol-grip like handle (8) formed to be grasped by the thumb and the first joint member of the index finger of a human hand and adapted for a holding of the implanting apparatus when placing the endoprothesis in the channel like vessel, which operating handle of the sleeve member (1) includes two projecting projections (10, 11) located diametrically opposite of each other which are formed as abutments for a human finger contacting the handle at one side and not surrounding same, and a further handle (9) of the core member (4) is located diametrically opposite of the pistol-grip shaped handle (8), whereby the two projections (10, 11) of the sleeve member (1) are located in a plain which coincides with the plain defined by the pistol-grip shaped handle (8) and the further handle (9).

2. Apparatus according to claim 1, characterized in that the further handle (9) is structured as an abutment for a human finger contacting the handle at one side and not surrounding same.

3. Apparatus according to claim 1 or 2, characterized in that the pistol-grip shaped handle (8) at the core member (4) is shaped to be surrounded and gripped by the middle, ring and little finger of a human body.

4. Apparatus according to one of the claims 1-3, characterized in that the core member (4) comprises at the proximal end at least one guide rod (12, 13) directed towards its distal end and which projects guided into a tube (14, 15) mounted firmly to the sleeve member (1) such to prevent a relative rotary motion between the sleeve member (1) and the core member (4).

5. Apparatus according to one of the claims 1-4, characterized in that the core member (4) and the sleeve member (1) consist of a rigid material.

6. Apparatus according to one of the claims 1-5, characterized in that the core member (4) is structured as a horton cylinder for the receipt of further medical instruments.

## Revendications

1. Instrument pour l'implantation d'une endoprothèse dans une cavité en forme de canal du corps humain ou animal, avec une coque en forme de douille tubulaire allongée (1) comprenant une extrémité distale (2) et une extrémité proximale (3), l'extrémité distale étant agencée pour recevoir une endoprothèse destinée à y être insérée sous une forme radialement comprimée, et avec un organe central formant un poussoir stationnaire (4) allongé, disposé dans la coque extérieure (1) coaxialement à celle-ci et déplaçable par rapport à elle, ce poussoir présentant une extrémité distale (5) et une extrémité proximale (6), la région de l'extrémité distale (5) comportant un épaulement (7) destiné à s'adosser à une endoprothèse qui y est insérée, de façon à ce qu'une endoprothèse introduite dans la coque (1) puisse en être expulsée par un mouvement relatif de la coque (1) par rapport à l'organe central (4), l'endoprothèse se dilatant ainsi d'elle-même et s'appliquant contre la paroi de la cavité, la coque (1) et l'organe central (4) possédant chacun à leur extrémité proximale une poignée de manoeuvre (3, respectivement 6), l'instrument possédant un dispositif (17) pour arrêter la coque extérieure par rapport à l'organe central dont la poignée de manoeuvre ressemble à une poignée protubérante de revolver (8) et est prévue pour être saisie par le pouce et la première phalange de l'index d'une main humaine afin de tenir l'instrument lors du placement de l'endoprothèse dans la cavité, la poignée de manoeuvre de la coque (1) comportant deux saillies (10, 11) diamétralement opposées qui protubèrent pour former appui pour un doigt qui y repose d'un côté sans entourer la poignée, une autre poignée (9) de l'organe central (4) étant diamétralement opposée à la poignée (8) ressemblant à une poignée de revolver, les deux saillies (10, 11) de la coque (1) étant situées dans un plan qui coïncide avec celui déterminé par l'autre poignée (9) et la poignée (8) ressemblant à une poignée de revolver.

2. Instrument selon la revendication 1, caractérisé en ce que l'autre poignée (9) est agencée pour former un appui pour un doigt agissant sur un côté et n'entourant pas la poignée.

3. Instrument selon la revendication 1 ou 2, caractérisé en ce que la poignée (8) ressemblant à une poignée de revolver sur l'organe central (4) est conformée pour être entourée par le majeur, l'annulaire et l'auriculaire d'une main humaine.

4. Instrument selon une des revendications 1 à 3, caractérisé en ce que l'organe central (4) comporte à son extrémité proximale au moins une tige de guidage (12, 13) dirigée vers son extrémité distale qui pénètre dans un tube (14, 15) fixé à demeure sur la coque (1) et y est guidée, afin d'éviter une rotation relative de la coque (1) par rapport à l'organe central (4).

5. Instrument selon une des revendications 1 à 4, caractérisé en ce que l'organe central (4) et la coque (1) sont réalisés en un matériau rigide.

6. Instrument selon une des revendications 1 à 5, caractérisé en ce que l'organe central (4) forme un cylindre creux pour contenir d'autres instruments médicaux.
